# EUROPEAN PATENT APPLICATION

(11) **EP 1 833 287 A2**
(43) Date of publication of application: **12.09.2007**
(21) Application number: 06077115.1
(22) Date of filing: 27.11.2006
(51) Int. Cl.: H05K 7/20

(54) **Heat radiating apparatus and optical projection device having the same**

(30) Priority: 07.03.2006 KR 20060021357
(71) Applicant: Samsung Electronics Co., Ltd., Suwon-si, Gyeonggi-Do (KR)
(72) Inventor: Kim, Sung-ki, Seocho-gu, Seoul (KR)
(74) Representative: Grootscholten, Johannes A.M.

(57) **Abstract**

A heat radiating apparatus and an optical projection device having the same. The heat radiating apparatus includes a cooling fluid circulating unit to circulate a cooling fluid via a heating part, and a heat exchanging unit to promote a heat exchange of the cooling fluid, which is circulated by the cooling fluid circulating unit, to an external air. The optical projection device includes at least one light emitting diode (LED) illumination system using a light emitting diode (LED) as a light source, and a radiating apparatus to radiate a heat generated from the light emitting diode (LED), wherein the radiating apparatus includes a cooling fluid circulating unit to circulate a cooling fluid via the light emitting diode (LED), and a heat exchanging unit to promote a heat exchange of the cooling fluid, which is circulated by the cooling fluid circulating unit, to an external air.

## Description

### BACKGROUND OF THE INVENTION

### 1. Field of the Invention

Apparatuses consistent with the present invention relate to a heat radiating apparatus and, more particularly, to a heat radiating apparatus using a cooling fluid that can effectively radiate a heat generated from an optical projection device using a light emitting diode (LED) as a light source, and an optical projection device having the heat radiating apparatus.

### 2. Description of the Related Art

Generally, an LED is advantageous in that a lifespan is guaranteed for a long time, there is no possibility of generating a harmful substance, and it is possible to freely form color coordinates. Accordingly, recently, the LED is in the spotlight as a light source for use in an illumination system in an optical projection device (e.g., a projector, a projection displaying device, etc.)

However, when the LED is used as the light source for the illumination system in an optical projection device, it is disadvantageous in that due to its high temperature heating characteristic, it is difficult to maintain a proper operation temperature, a lifespan is reduced and a color transformation is generated. Accordingly, there is a need for a heat radiating apparatus that can effectively radiate a heat generating from the LED while the LED is operated.

A heat pipe type heat radiating apparatus is widely used as a heat radiating apparatus for the projection device using an LED as the light source. The heat pipe type heat radiating apparatus includes a heat pipe connected to the LED to radiate a heat generated therefrom.

The conventional heat radiating apparatus described as above, however, presents a problem that a heat generated from the LED of high brightness and high temperature is not effectively radiated due to the limitation of radiating the heat with the heat pipe.

Also, the conventional heat radiating apparatus presents a problem in that the heat generated from the LED travels into the optical projection device to increase an internal temperature thereof, thereby resulting in abnormal operation of the optical projection device.

### SUMMARY OF THE INVENTION

Illustrative, non-limiting embodiments of the present invention overcome the above disadvantages and other disadvantages not described above. Also, the present invention is not required to overcome the disadvantages described above, and an illustrative, non-limiting embodiment of the present invention may not overcome any of the problems described above.

Accordingly, an aspect of the present invention is to provide a heat radiating apparatus using a cooling fluid that can effectively radiate a heat generated from an optical projection device using a light emitting diode (LED) as a light source, and an optical projection device having the heat radiating apparatus.

Another aspect of the present invention is to provide a heat radiating apparatus that an entry of heat generated from the heat radiating apparatus and/or a heating part into a device to which the heat radiating apparatus is applied is prevented, thereby preventing an internal temperature of the device from being increased, and an optical projection device having the heat radiating apparatus.

According to one aspect of an exemplary embodiment of the present invention, there is provided a heat radiating apparatus comprising a cooling fluid circulating unit to circulate a cooling fluid via a heating part, and a heat exchanging unit to promote a heat exchange of the cooling fluid, which is circulated by the cooling fluid circulating unit, to an external air.

The cooling fluid circulating unit may comprise a radiating plate having a plurality of cooling fluid circulating lines formed therein, the radiating plate being in contact with the heating part, a circulating piping connected to the cooling fluid circulating lines of the radiating plate to constitute a closed loop, and a pump to circulate the cooling fluid in the circulating piping.

The heat exchanging unit may comprise at least one heat exchanging unit disposed on the circulating piping.

The heat exchanging unit may comprise a plurality of radiating fins arranged on the circulating piping.

The heat exchanging unit may further comprise a fan unit to induce a flow of the external air to the radiating fins.

Alternatively, the heat radiating apparatus may further comprise a housing to receive the cooling fluid circulating unit and the heat exchanging unit, the housing having an inlet and an outlet.

Fans may be disposed respectively at the inlet and the outlet of the housing.

The heating part may use a light emitting diode (LED) as a light source.

According to another aspect of an exemplary embodiment of the present invention, there is provided an optical projection device comprising at least one light emitting diode (LED) illumination system using a light emitting diode (LED) as a light source, and a heat radiating apparatus to radiate a heat generated from the light emitting diode (LED), wherein the heat radiating apparatus comprises a cooling fluid circulating unit to circulate a cooling fluid via the light emitting diode (LED), and a heat exchanging unit to promote a heat exchange of the cooling fluid, which is circulated by the cooling fluid circulating unit, to an external air.

The cooling fluid circulating unit may comprise a radiating plate having a plurality of cooling fluid circulating lines formed therein, the radiating plate being in contact with the light emitting diode (LED), a circulating piping connected to the cooling fluid circulating lines of the radiating plate to constitute a closed loop, and a pump to circulate the cooling fluid in the circulating piping.

The heat exchanging unit may comprise a plurality of radiating fins arranged in a spaced-apart relation with each other on the circulating piping.

The heat exchanging unit may further comprise a fan unit to induce a flow of the external air to the radiating fins.

Alternatively, the optical projection device may further comprise a housing configured in a shape to receive the cooling fluid circulating unit and the heat exchanging unit,

The housing may comprises an inlet and an outlet.

The fan unit may comprise a suction fan disposed at the inlet, and a discharging fan disposed at the outlet.

Other objects, advantages, and salient features of the invention will become apparent to those skilled in the art from the following detailed description, which, taken in conjunction with the accompanying drawings, discloses exemplary embodiments of the invention.

### BRIEF DESCRIPTION OF THE DRAWINGS

These and/or other aspects and advantages of exemplary embodiments of the present invention will become apparent and more readily appreciated from the following description of the exemplary embodiments, taken in conjunction with the accompanying drawings, of which:
FIG. 1 is a perspective view schematically illustrating a heat radiating apparatus in accordance with an exemplary embodiment of the present invention; and
FIG. 2 is a perspective view an optical projection device with another exemplary embodiment of the present invention to which the heat radiating apparatus of FIG. 1 is applied.

### DETAILED DESCRIPTION OF THE EXEMPLARY EMBODIMENTS

Reference will now be made in detail to the exemplary embodiments of the present invention, examples of which are illustrated in the accompanying drawings, wherein like reference numerals refer to the like elements throughout. The exemplary embodiments are described below in order to explain the present invention by referring to the figures.

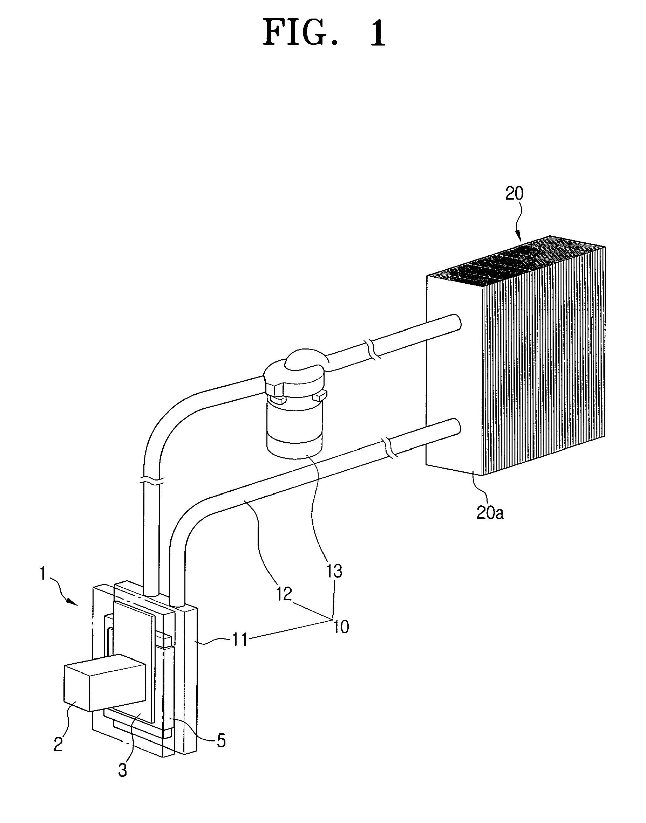

FIG. 1 is a perspective view schematically illustrating a heat radiating apparatus in accordance with the exemplary embodiment of the present invention for use in an optical projection device, such as a projector and a projection displaying device.

As shown in FIG. 1, the heat radiating apparatus in accordance with the exemplary embodiment of the present invention includes a cooling fluid circulating unit 10 to circulate a cooling fluid via a heating part 1, and a heat exchanging unit 20 to promote a heat exchange of the cooling fluid, which is circulated by the cooling fluid circulating unit 10, to an external air. Here, the heating part 1 may be a part having a light emitting diode (LED) 2 as a heating element. The LED 2 is used as a light source in the optical projection device such as a projector or a projection displaying device, and is mounted on a printed circuit board (PCB) 3 for controlling an operation of the LED 2.

The cooling fluid circulating unit 10 is provided with a radiating plate 11, a circulating piping 12, and a pump 13. The radiating plate 11 is in contact with the heating part 1, and radiates heat, which is generated from the LED 2. In order to transmit the heat generated from the LED 2 to the radiating plate 11, a heat conductor 5 is disposed between the PCB 3 and the radiating plate 11. In the radiating plate 11, a plurality of cooling fluid circulating lines (not shown) are formed. The circulating piping 12 is connected to the cooling fluid circulating lines of the radiating plate 11 to constitute a closed loop. The pump 13 is disposed in a fluid path of the circulating piping 12 to circulate the cooling fluid in the circulating piping 12. Although a single pump 13 is illustrated as disposed at a given location in the circulating piping 12, a plurality of pumps (not shown) can be disposed at corresponding respective locations along the fluid path of the circulating piping 12.

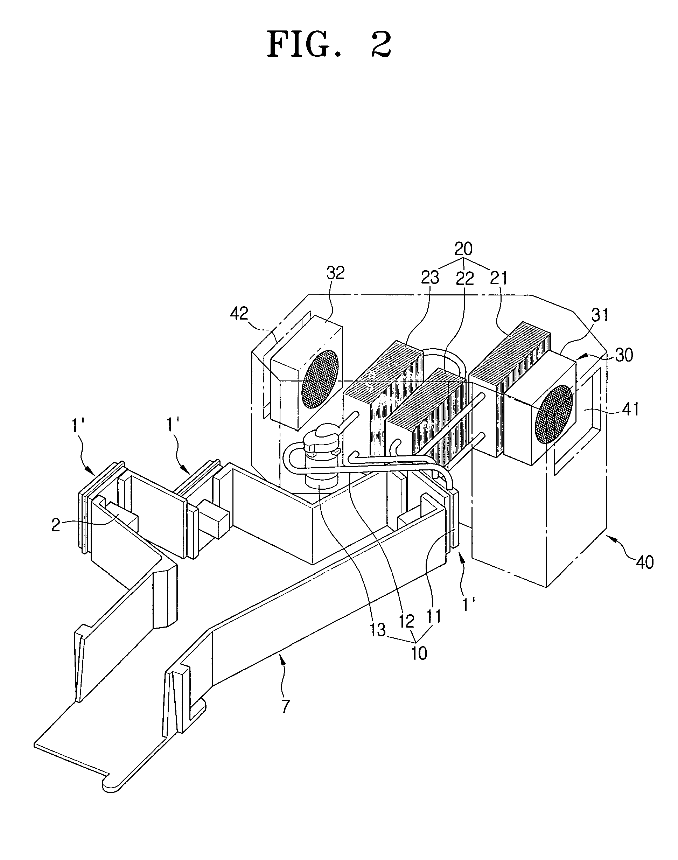

The heat exchanging unit 20 is disposed on the circulating piping 12. The heat exchanging unit 20 may be formed of more than one, e.g., three heat exchanging units 21, 22 and 23 (see FIG. 2). The heat exchanging unit 20 includes a plurality of radiating fins 20a, which are arranged at a regularly or irregularly spaced-apart relation with each other on the circulating piping 12. The radiating fins 20a may be formed of a metal plate such as, for example, a thin aluminum plate, and are disposed vertically to the circulating piping 12 in an orientation which allows hot air to flow smoothly through the heat radiating apparatus by convection. In addition, the heat exchanging unit 20 may further include a fan unit 30 (see FIG. 2) to induce a flow of the external air to the radiating fins 20a.

FIG. 2 is a perspective view schematically illustrating an optical projection device, such as a projector or a projection displaying device, in accordance with another exemplary embodiment of the present invention, to which the heat radiating apparatus of FIG. 1 is applied.

As shown in FIG. 2, the optical projection device in accordance with another exemplary embodiment of the present invention includes three LED illumination systems 1 each using an LED 2 as a light source, and a heat radiating apparatus to radiate a heat generated from the LED 2.

The radiating apparatus includes a cooling fluid circulating unit 10, a heat exchanging unit 20, a fan unit 30, and a housing 40. Here, since the structure of the cooling fluid circulating unit 10, the heat exchanging unit 20, and the fan unit 30 except for the housing 40 is the same as that of the radiating apparatus explained with reference to FIG. 1, detailed descriptions and illustrations thereof are omitted.

The housing 40 is configured, so that it receives the cooling fluid circulating unit 10, the heat exchanging unit 20, and the fan unit 30 therein. The housing 40 has an air inlet 41 and an air outlet 42 formed at opposite sidewalls, respectively. A suction fan 31 for drawing in air is disposed at the air inlet 41, and a discharging fan 32 for discharging air is disposed at the air outlet 42. The suction fan 31 and the discharging fan 32 constitute the fan unit 30.

Hereinafter, an operation of the heat radiating apparatus constructed as above will now be described in detail.

First, a heat generated from the LED 2 is transmitted to the heat conductor 5 through the PCB 3, and then radiated to the radiating plate 11. The heat radiated to the radiating plate 11 is cooled by a cooling fluid, which circulates through the circulating piping 12 connected to the cooling fluid circulating lines in the radiating plate 11. While passing by the LED 2, the cooling fluid is heated, so that a temperature thereof is increased. The heated cooling fluid is moved to the heat exchanging unit 20 through the circulating piping 12. As the heated cooling fluid is moved to the heat exchanging unit 20, it radiates an internal heat to the outside through the plurality of radiating fins 20a, so that the temperature thereof is lowered. And then, the cooling fluid in which the temperature is lowered is again circulated via the LED 2 by the pump 13. As a result, the heat generated from the LED 2 can be effectively radiated.

As described above, the heat radiating apparatus in accordance with the exemplary embodiment of the present invention effectively radiates the heat generated from the LED 2 by using the cooling fluid. Accordingly, a predetermined lifespan and a stable operation of the LED 2 can be guaranteed. In addition, the heat radiating apparatus in accordance with the exemplary embodiment of the present invention induces the flow of the external air to the radiating fins 20a with the fan unit 30. Accordingly, the heat exchange of the cooling fluid can be maximized.

In the optical projection device in accordance with the another exemplary embodiment of the present invention to which the heat radiating apparatus is applied, the heat radiating apparatus further includes the housing 40 to receive the cooling fluid circulating unit 10, the heat exchanging unit 20 and the like. Accordingly, the heat, which is radiated through the cooling fluid circulating unit 10, the heat exchanging unit 20 and the like, is blocked by the housing 40, so that the entry of the heat into an internal unit 7 including the LED illumination systems 1 vulnerable to heat is prevented, thereby preventing a temperature of the internal unit 7 from being increased.

According to the exemplary embodiments of the present invention as described above, the heat radiating apparatus and the optical projection device having the same can effectively cool and radiate the heat generated from the optical projection device using the LED as a light source.

Also, according to the exemplary embodiments of the present invention, the heat radiating apparatus and the optical projection device having the same can prevent the entry of heat generated from the heat radiating apparatus and/or the LED into the internal unit of the optical projection device, and thereby prevent the temperature of the internal unit from being increased.

Although a few exemplary embodiments of the present invention have been shown and described, it will be appreciated by those skilled in the art that changes may be made in these embodiments without departing from the principles and spirit of the invention, the scope of which is defined in the appended claims and their equivalents.

## Claims

1. A heat radiating apparatus comprising:
a cooling fluid circulating unit to circulate a cooling fluid via a heating part; and
a heat exchanging unit which communicates with the cooling fluid circulating unit to promote a heat exchange of the circulated cooling fluid to an external air.

2. The heat radiating apparatus as claimed in claim 1, wherein the cooling fluid circulating unit comprises:
a radiating plate having a plurality of cooling fluid circulating lines formed therein, the radiating plate being in contact with the heating part;
a circulating piping connected to the cooling fluid circulating lines of the radiating plate to constitute a closed loop; and
a pump disposed in a fluid path of the circulating piping to circulate the cooling fluid in the circulating piping.

3. The heat radiating apparatus as claimed in claim 2, wherein the heat exchanging unit comprises at least one heat exchanging unit disposed on the circulating piping.

4. The heat radiating apparatus as claimed in claim 3, wherein the heat exchanging unit comprises a plurality of radiating fins arranged on the circulating piping.

5. The heat radiating apparatus as claimed in claim 4, wherein the heat exchanging unit further comprises a fan unit to induce a flow of the external air to the radiating fins.

6. The heat radiating apparatus as claimed in claim 1, further comprising a:
housing to receive the cooling fluid circulating unit and the heat exchanging unit, the housing having an inlet and an outlet.

7. The heat radiating apparatus as claimed in claim 6, wherein fans are disposed respectively at the inlet and the outlet of the housing.

8. The heat radiating apparatus as claimed in claim 1, wherein the heating part comprises:
a light emitting diode (LED); and
a printed circuit board (PCB) on which the light emitting diode (LED) is mounted.

9. An optical projection device comprising:
at least one light emitting diode (LED) illumination system using a light emitting diode (LED) as a light source; and
a heat radiating apparatus to radiate a heat generated from the light emitting diode (LED),
wherein the heat radiating apparatus comprises a cooling fluid circulating unit to circulate a cooling fluid so as to cool a printed circuit board (PCB) on which the light emitting diode (LED) is mounted, and a heat exchanging unit to promote a heat exchange of the cooling fluid, which is circulated by the cooling fluid circulating unit, to an external air.

10. The optical projection device as claimed in claim 9, wherein the cooling fluid circulating unit comprises:
a radiating plate having a plurality of cooling fluid circulating lines formed therein to cool the heat transmitted from the printed circuit board (PCB);
a circulating piping connected to the cooling fluid circulating lines of the radiating plate to constitute a closed loop; and
a pump to circulate the cooling fluid in the circulating piping.

11. The optical projection device as claimed in claim 10, wherein the heat
exchanging unit comprises at least one heat exchanging unit disposed on the circulating piping.

12. The optical projection device as claimed in claim 11, wherein the heat exchanging unit comprises a plurality of radiating fins arranged in a spaced-apart relation with each other on the circulating piping.

13. The optical projection device as claimed in claim 12, wherein the heat exchanging unit further comprises a fan unit to induce a flow of the external air to the radiating fins.

14. The optical projection device as claimed in claim 10,
further comprising a heat conductor disposed between the printed circuit board (PCB) and the radiating plate,
wherein the radiating plate receives the heat of the printed circuit board (PCB) through the heat conductor.

15. The optical projection device as claimed in claim 9, further comprising a housing configured in a shape to receive the cooling fluid circulating unit and the heat exchanging unit.

16. The optical projection device as claimed in claim 15, wherein the housing comprises an inlet and an outlet.

17. The optical projection device as claimed in claim 16, further comprising:
a suction fan disposed at the inlet; and
a discharging fan disposed at the outlet.
